(19) Europäisches Patentamt
European Patent Office
Office européen des brevets

(11) EP 0 581 082 B1

(12) **EUROPÄISCHE PATENTSCHRIFT**

(45) Veröffentlichungstag und Bekanntmachung des Hinweises auf die Patenterteilung:
**26.02.1997 Patentblatt 1997/09**

(51) Int Cl.[6]: **C07F 7/08**, C07C 213/04, C07C 303/32, C08G 77/38

(21) Anmeldenummer: **93111003.5**

(22) Anmeldetag: **09.07.1993**

(54) **Verfahren zur Herstellung von tensidischen Anion-Kation-Komplexen**

Process for the preparation of surface-active anion-cation complexes

Procédé de préparation de complexes anion-cation tensio-actifs

(84) Benannte Vertragsstaaten:
**BE DE FR GB NL**

(30) Priorität: **22.07.1992 DE 4224136**

(43) Veröffentlichungstag der Anmeldung:
**02.02.1994 Patentblatt 1994/05**

(73) Patentinhaber: **Th. Goldschmidt AG**
**D-45127 Essen (DE)**

(72) Erfinder:
- **Koerner, Götz, Dr.**
  **D-45259 Essen (DE)**
- **Klein, Klaus-Dieter, Dr.**
  **D-45468 Mülheim a.d. Ruhr (DE)**
- **Schaefer, Dietmar, Dr.**
  **D-45529 Hattingen (DE)**

(56) Entgegenhaltungen:
**EP-A- 0 017 121**
**EP-A- 0 129 158**
**EP-A- 0 364 708**
**DE-A- 2 852 431**
**DE-B- 1 150 095**
**US-A- 2 810 747**
**US-A- 2 968 664**
**US-A- 3 168 546**
**US-A- 3 389 160**
**US-A- 3 507 897**
**US-A- 4 093 642**
**US-A- 4 514 547**
**US-A- 5 068 380**

- **S. Riethmayer "Antistatika" Gummi Asbest Kunststoffe 5/1973, Seiten 419 - 429**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft ein Verfahren zur Herstellung von tensidischen Anion-Kation-Komplexen. Die Erfindung betrifft insbesondere ein einstufiges Verfahren zur Herstellung von tensidischen Anion-Kation-Komplexen, bei denen bevorzugt die anionische und/oder kationische Komponente eine siliciumorganische Verbindung ist.

Anion-Kation-Komplexe mit im wesentlichen pH-unabhängigen tensidischen Eigenschaften sind in der US-PS 4 093 642 beschrieben. Diese Komplexe enthalten mindestens eine siliciumorganische Verbindung, die als Siloxan A oder B definiert ist.

Das Siloxan A weist die Strukturformel

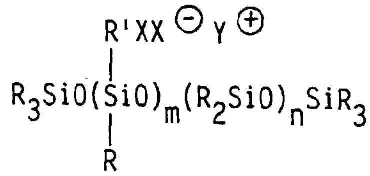

auf, wobei

- R eine einwertige Kohlenwasserstoffgruppe mit 1 bis 18 C-Atomen ist,
- R' eine zweiwertige organische Gruppe mit 1 bis 18 C-Atomen ist,
- $XX^{\ominus}$ eine zweiwertige anionische Gruppe ist, welche an R' kovalent und an $Y^{\oplus}$ ionisch gebunden ist,
- $Y^{\oplus}$ ein einwertiges Kation ist, welches wenigstens 8 C-Atome aufweist, und von einem kationischen Tensid mit einem Halogen-Gegenion durch Entfernen des Halogens hergeleitet ist, und welches frei von ionisch gebundenem Wasserstoff ist,
- m eine ganze Zahl mit einem Wert von 1 bis 100 ist,
- n eine ganze Zahl mit einem Wert von 0 bis 200 ist, und das Verhältnis m / (n+2) 0,1 bis 20 beträgt.

Das Siloxan B weist die Strukturformel

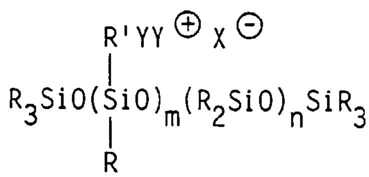

auf, wobei

- R eine einwertige Kohlenwasserstoffgruppe mit 1 bis 18 C-Atomen ist,
- R' eine zweiwertige organische Gruppe mit 1 bis 18 C-Atomen ist,
- $YY^{\oplus}$ eine zweiwertige kationische Gruppe ist, welche an R' kovalent und an $X^{\ominus}$ ionisch gebunden und frei von ionisch gebundenem Wasserstoff ist,
- $X^{\ominus}$ ein einwertiges Anion ist, welches wenigstens 8 C-Atome aufweist, und von einem anionischen Tensid mit einem einwertigen Metall-Gegenion durch Entfernen des Metalls hergeleitet ist,
- m eine ganze Zahl mit einem Wert von 1 bis 100 ist,
- n eine ganze Zahl mit einem Wert von 0 bis 200 ist, und das Verhältnis m / (n+2) 0,1 bis 20 beträgt.

Die Herstellung der tensidischen Anion-Kation-Komplexe geschieht nach dieser US-PS 4 093 642 in der Weise, daß man zunächst getrennt das anionische Tensid und das kationische Tensid herstellt und dann äquimolare Mengen beider Tenside in einem Lösungsmittel, wie Wasser oder Alkohol, vermischt und, gegegebenfalls nach Entfernen des Lösungsmittels, das ausgefallene Salz abtrennt.

Für den Fall, daß beide Tensidkomponenten siliciumorganische Verbindungen sind, kann man diese Reaktion wie folgt beschreiben:

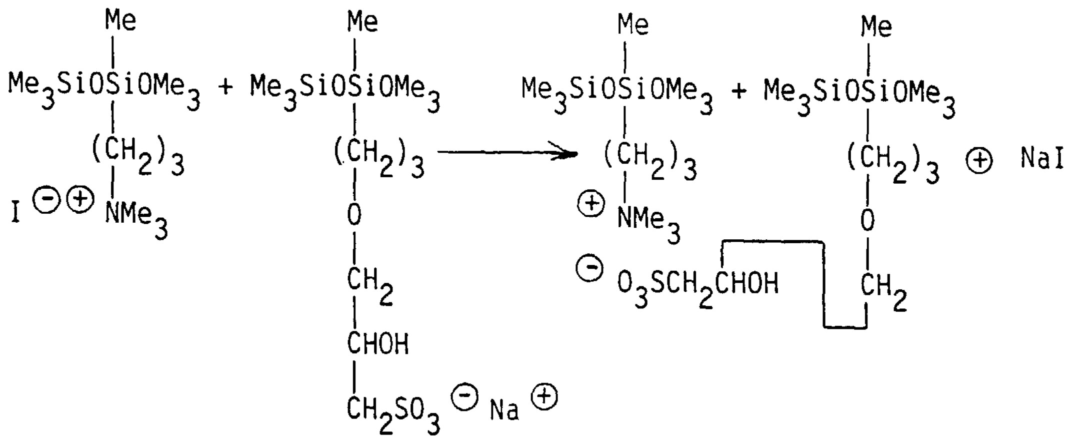

Kation-Tensid Anion-Tensid Kation-Anion-Komplex

Anstelle eines Silicontensides kann auch ein kohlenstofforganisches Tensid ausgewählt werden.

Die Tensidkomplexe weisen hohe Oberflächenaktivität auf und sind vielseitig verwendbar. Es ist aber nachteilig, daß man entsprechend diesem Stand der Technik ein zweistufiges Verfahren durchlaufen und eine erhebliche Salzfracht entfernen muß. Das Verfahren ist auch nicht geeignet, längerkettige und polyfunktionelle Silieonsulfonate und deren Kation-Komplexe herzustellen.

Die vorliegende Erfindung befaßt sich deshalb insbesondere mit der Vereinfachung der Herstellung solcher Komplexe, wobei ein einstufiges Verfahren angestrebt wird, das auch auf längerkettige und polyfunktionelle Siloxane angewendet werden kann.

Gegenstand der Erfindung ist deshalb ein Verfahren zur Herstellung von tensidischen Anion-Kation-Komplexen. Die Erfindung betrifft insbesondere ein einstufiges Verfahren zur Herstellung von tensidischen Anion-Kation-Komplexen, bei denen bevorzugt die anionische und/oder kationische Komponente eine siliciumorganische Verbindung ist, mit dem Kennzeichen, daß man eine kohlenstoff- oder siliciumorganische Verbindung mit mindestens einer Epoxidgruppe mit einem quaternären Ammoniumhydrogensulfit der allgemeinen Formel

$$HN^{\oplus}(R^1)_3SO_3H^{\ominus}, \qquad I$$

wobei
$R^1$ innerhalb des Moleküls gleich oder verschieden ist und

(1) ein Alkyl- oder Hydroxyalkylrest mit 1 bis 18 Kohlenstoffatomen,
(2) ein Phenylrest oder
(3) ein Rest der Formel $C_nH_{2n+1}CONH\text{-}(CH_2)_m\text{-}$, in der n eine Zahl von 7 bis 17 und m = 2 oder 3 ist, sein kann,

in Gegenwart eines polaren Lösungsmittels in solchen Mengen umsetzt, daß das Molverhältnis Epoxidgruppe : $HN^{\oplus}(R^1)_3SO_3H^{\ominus}$ ≥ 1 : 1 beträgt.

Die Reaktion verläuft chemisch in folgender Weise

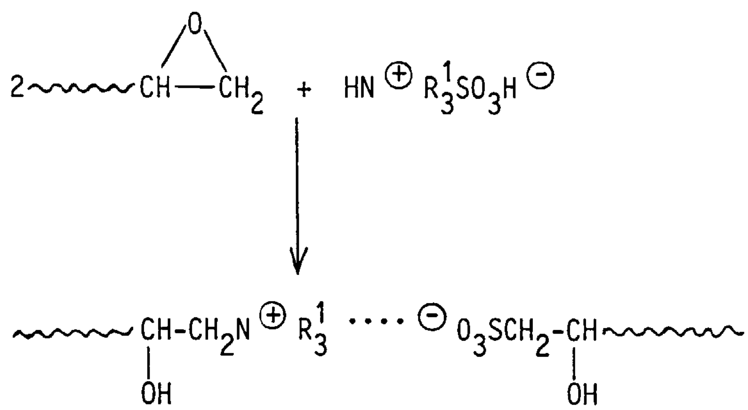

Kation-Anion-Komplex

Bei dem erfindungsgemäßen Verfahren entstehen keine abzutrennenden Nebenprodukte.

Das erfindungsgemäße Verfahren wird in Gegenwart eines polaren Lösungsmittels, vorzugsweise eines niedrigsiedenden, aliphatischen Alkohols, insbesondere bevorzugt in Gegenwart von Isopropanol, durchgeführt.

Hat $R^1$ die Bedeutung eines Alkyl- oder Hydroxyalkylrestes, können die Reste geradkettig oder verzweigt sein. Bevorzugt sind die geradkettigen Reste, wie der Methyl-, Ethyl-, Propyl-, Butyl-, Hexyl-, Octyl-, Decyl-, Dodecyl- oder Octadecylrest und die entsprechenden Hydroxyalkylreste. Durch die Auswahl der Reste $R^1$ können die tensidischen Eigenschaften der Komplexe verändert werden, da mit steigender Kohlenstoffanzahl die Hydrophilie des Komplexes abnimmt.

$R^1$ kann die Bedeutung eines Phenylrestes haben. $R^1$ kann aber auch ein Rest der Formel $C_nH_{2n+1}CONH\text{-}(CH_2)_m\text{-}$ sein, in der n eine Zahl von 7 bis 17 und m = 2 oder 3 ist. Der Rest $C_nH_{2n+1}CO$ ist dabei von einer Fettsäure abgeleitet, wie z.B. von der Laurin-, Palmitin- oder Stearinsäure. Dieser Acylrest kann aber auch von einem Fettsäuregemisch abgeleitet sein, wie es beispielsweise in der Kokosfettsäure vorliegt.

Die Reste $R^1$ können in dem quaternären Ammoniumhydrogensulfit unterschiedliche Bedeutung haben. So können am gleichen Stickstoffatom z.B. zwei Methylgruppen und eine Gruppe $C_{12}H_{25}CONH\text{-}(CH_2)_3\text{-}$ gebunden sein. Weitere Beispiele geeigneter und zugleich bevorzugter quaternärer Ammoniumhydrogensulfite sind Triethylammoniumhydrogensulfit, (2-Hydroxyethyl)-dimethylammoniumhydrogensulfit und (2-Hydroxypropyl)dimethylammoniumhydrogensulfit. Es sind jedoch im Prinzip beliebige Kombinationen möglich, so daß man die Wahl der Reste $R^1$ im Hinblick auf den gewünschten Anwendungszweck treffen kann.

Als kohlenstofforganische Verbindungen mit mindestens einer Epoxidgruppe sind die Epoxyalkane geeignet. Dabei kann die Kohlenstoffkette des Epoxyalkans verzweigt sein. Vorzugsweise werden als Epoxyalkane Verbindungen der allgemeinen Formel

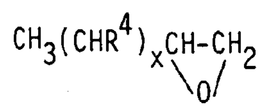

verwendet. In dieser Formel bedeutet $R^4$ einen Wasserstoff- oder einen Alkylrest mit 2 bis 6 Kohlenstoffatomen, der innerhalb des Moleküls gleich oder verschieden sein kann. Hat x einen Wert von 5, kann $R^4$ beispielsweise einmal die Bedeutung eines Alkylrestes mit 2 bis 6 Kohlenstoffatomen und viermal die Bedeutung eines Wasserstoffrestes haben. x hat einen Wert von 2 bis 14.

Vorzugsweise hat in dem Epoxyalkan $R^4$ die Bedeutung eines Wasserstoffrestes. Im Falle der Verzweigung der Kohlenstoffkette hat vorzugsweise nur ein Rest $R^4$ die Bedeutung eines Alkylrestes. Besonders bevorzugte Verbindungen sind 1,2-Epoxyoctan, 1,2-Epoxydodecan und 1,2-Epoxyhexadecan. Als Epoxidgruppen aufweisende Verbindungen kann man auch die Epoxide ungesättigter Fettsäuren oder deren Ester einsetzen. Beispiele hierfür sind die epoxidierte Linol- und Linolensäure und deren Ester.

Siliciumorganische Verbindungen mit mindestens einer Epoxidgruppe entsprechen vorzugsweise der allgemeinen durchschnittlichen Formel

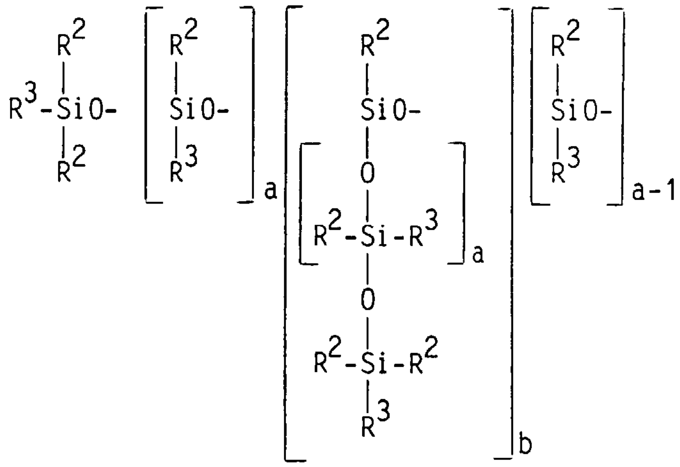

II

wobei

$R^2$ im durchschnittlichen Molekül gleich oder verschieden ist und Alkylreste mit 1 bis 18 Kohlenstoffatomen bedeutet, jedoch mindestens 90 % der Reste $R^2$ Methylreste sind,

$R^3$ die Bedeutung des Restes $R^2$ hat oder ein über Kohlenstoff an Silicium gebundener Epoxidrest ist, mit der Maßgabe, daß im durchschnittlichen Molekül mindestens ein Rest $R^3$ ein Epoxidrest ist,

a einen Wert von 0 bis 500 und

b einen Wert von 0 bis 5 hat.

Wie oben erwähnt, handelt es sich bei dieser Formel um eine durchschnittliche allgemeine Formel. Sie gibt insbesondere die Struktureinheiten und ihre Anzahl innerhalb des Moleküls an. Es ist jedoch zu beachten, daß es sich in den meisten Fällen um polymere siliciumorganische Verbindungen handelt, deren Kettenlängen und Verzweigungsgrad unterschiedlich sein können, aber im Durchschnitt den angegebenen Werten entsprechen.

Der Index a hat einen Wert von 0 bis 500, vorzugsweise einen Wert von 1 bis 200 und besonders bevorzugt einen Wert von 1 bis 100.

Der Index b hat einen Wert von 0 bis 5, bevorzugt 0 bis 3. Hat b den Wert 0 und a den Wert 1, ist die siliciumorganische Verbindung ein modifiziertes Trisiloxan. Mit steigendem Wert von a nimmt die Kettenlänge, mit steigendem Wert von b der Verzweigungsgrad zu.

Mindestens ein Rest $R^3$ muß in der Formel II die Bedeutung eines Epoxidrestes haben. Bevorzugte Beispiele geeigneter Epoxidreste sind solche der Formeln

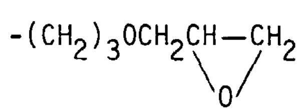 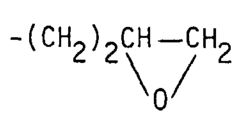

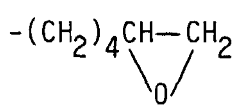 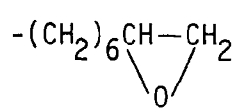

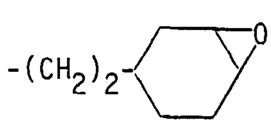 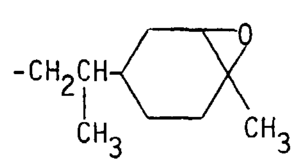 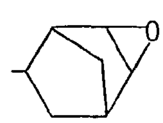

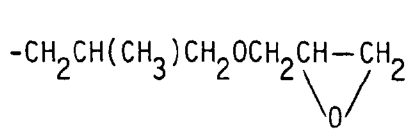

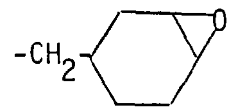

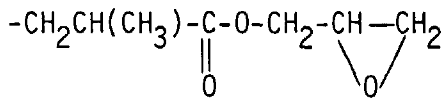

Das erfindungsgemäße Verfahren kann in der Weise durchgeführt werden, daß man eine siliciumorganische Verbindung der Formel II oder ein Gemisch hiervon mit dem quaternären Ammoniumhydrogensulfit umsetzt oder daß man ein Gemisch von siliciumorganischen Verbindungen der Formel II und kohlenstofforganischen Verbindungen mit jeweils mindestens einer Epoxidgruppe verwendet.

Vorzugsweise setzt man als Verbindung mit mindestens einer Epoxidgruppe siliciumorganische Verbindungen der Formel II ein. Man hat aber die Möglichkeit, durch die gleichzeitige Anwesenheit von kohlenstofforganischen Verbindungen mit mindestens einer Epoxidgruppe die Eigenschaften der tensidischen Anion-Kation-Komplexe zu beeinflussen.

Beispiele von erfindungsgemäß hergestellten tensidischen Anion-Kation-Komplexen sind

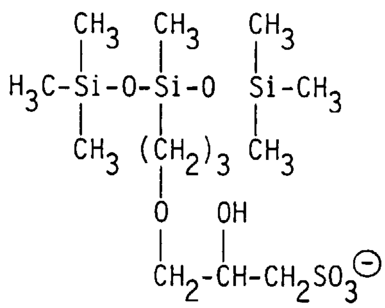

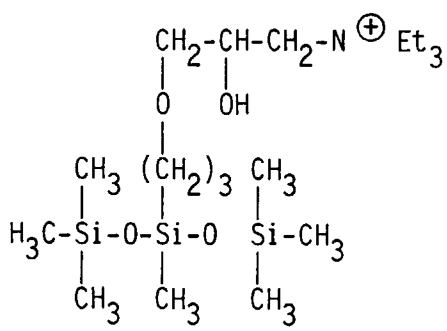

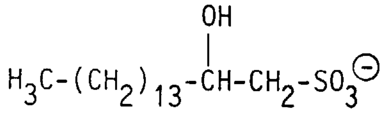

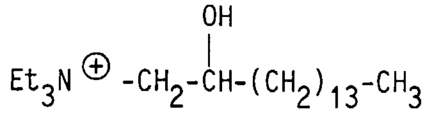

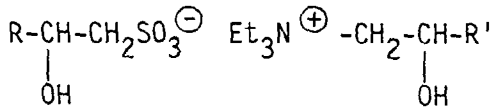

R = $H_3C(CH_2)_{13}$- und

R' = 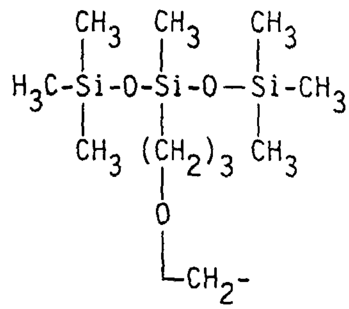

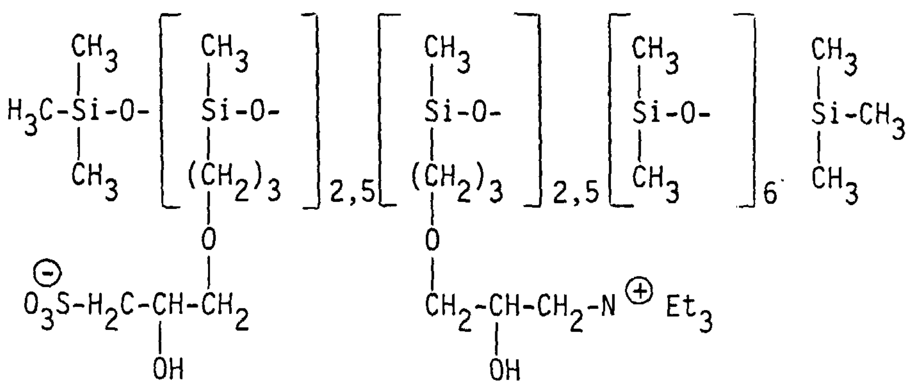

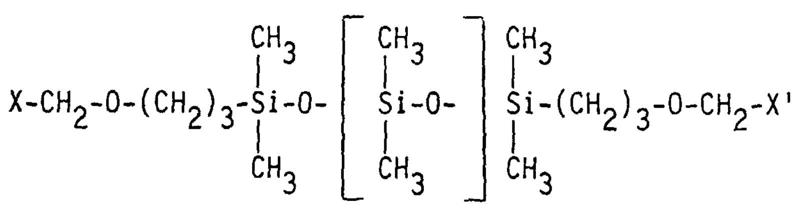

X = 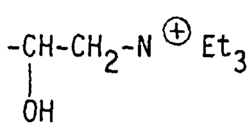 und X' = 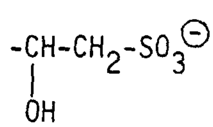

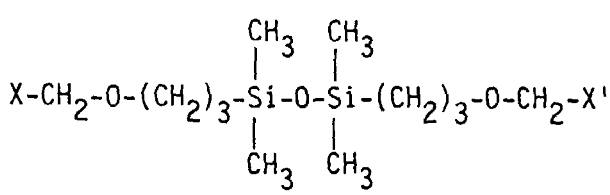

X und X' wie oben definiert

Die erfindungsgemäß hergestellten Komplexe zeichnen sich durch eine kleine kritische Micellkonzentration [cmc] aus. Bei der Umsetzung von mit Epoxiden modifizierten Organopolysiloxanen erhält man viskose, klare Flüssigkeiten. Mit steigendem Zusatz von Alkanepoxiden zum Reaktionsgemisch erhält man in Abhängigkeit von der Alkylkettenlänge meist wachsartige Festkörper mit definierten Schmelzpunkten.

Die Eigenschaften der erfindungsgemäß hergestellten Anion-Kation-Komplexe ergeben sich in dem Fachmann verständlicher Weise je nach Art der gewählten Edukte. Ist das Gerüst des Polysiloxans kurzkettig, überwiegen die typischen Eigenschaften eines Netzmittels. Mit steigendem Molekulargewicht und gegebenenfalls mit Verwendung von Alkanepoxiden steigt die relative Verträglichkeit dieser Produkte mit Kunststoffen. Die Produkte entfalten dann antistatische, schmierende oder auch trennende Eigenschaften. Die erfindungsgemäß hergestellten Komplexe können auch als Emulgatoren, insbesondere zur Herstellung von W/O-Emulsionen eingesetzt werden.

In den folgenden Beispielen 2 bis 12 wird das erfindungsgemäße Verfahren näher erläutert, und es werden anwendungstechnische Eigenschaften der Verfahrensprodukte gezeigt. Das Beispiel 1 beschreibt die Herstellung des bei der Reaktion verwendeten quaternären Ammoniumhydrogensulfits. Diese Herstellung erfolgt in an sich bekannter Weise. Das Beispiel 1 ist deshalb nicht erfindungsgemäß.

Beispiel 1

(nicht erfindungsgemäß)

Herstellung von Triethylammoniumhydrogensulfit nach an sich bekannten Verfahren

101,19 g (1 Mol) Triethylamin, 18,0 g (1 Mol) Wasser und 200,0 g Isopropanol werden in einem 500-ml-Dreihalskolben, versehen mit Rückflußkühler, Rührer, Gaseinleitungsrohr und Innenthermometer, unter Rühren vermischt. Dann wird bei Raumtemperatur Schwefeldioxid eingeleitet, wobei die Innentemperatur bis auf 42°C ansteigt. Nach Abklingen der Exothermie wird die Gaszufuhr abgebrochen und durch Auswiegen nach Durchleiten von Stickstoff die Schwefeldioxidaufnahme bestimmt.

Theorie: 64,0 g Massezuwachs; Praxis: 64,3 g

Das Produkt wird ohne weitere Aufarbeitung weiterverwendet. (2-Hydroxypropyl)-dimethylammoniumhydrogensulfit und (2-Hydroxyethyl)-dimethylhydrogensulfit werden in gleicher Weise dargestellt.

Herstellung der erfindungsgemäßen Verbindungen

Beispiel 2

Umsetzung von 3-(Glycidyloxypropyl)-1,1,1,3,5,5,5-heptamethyltrisiloxan mit Triethylammoniumhydrogensulfit

In einem 2-l-Dreihalskolben, ausgestattet mit Rührer, Rückflußkühler und Tropftrichter, werden zu 183,5 g (1 Mol) Triethylammoniumhydrogensulfit, gelöst in 200,0 g Isopropanol, 627,4 g (2 Val) 3-(Glycidyloxypropyl)-1,1,1,3,5,5,5-heptamethyltrisiloxan bei einer Innentemperatur von 30°C zugetropft. Dann rührt man 1 h nach, wobei sich die Reaktionsmischung klärt, und erhitzt anschließend 6 h auf Rückflußtemperatur.

Nach dem Abkühlen wird mit 1 Gew.-% Bentonit als Filterhilfe versetzt und filtriert. Danach wird das Produkt im ölpumpenvakuum bei 80°C Wasserbadtemperatur von flüchtigen Bestandteilen befreit.

Man erhält ein wasserklares, viskoses Produkt [Viskosität (24°C, nach Brookfield) = 18 550 mPa.s], das sich trübe in Wasser löst.

Die 1 %ige Lösung in destilliertem Wasser weist eine Oberflächenspannung von 20,7 mN/m auf und ergibt auf einer Polypropylenplatte eine Spreitung von 65 mm. Analytische Untersuchungen mittels $^{1}H$- und $^{13}C$-NMR-Spektroskopie bestätigen, daß das Umsetzungsprodukt die erwartete Struktur

$$Me_3SiO(SiMeO)SiMe_3$$
$$(CH_2)_3OCH_2(CHOH)CH_2N^{\oplus}Et_3$$
$$(CH_2)_3OCH_2(CHOH)CH_2SO_3^{\ominus}$$
$$Me_3SiO(SiMeO)SiMe_3$$

besitzt.

Das Produkt ist mit einem hydroxyfunktionellen Polydimethylsiloxan der mittleren Kettenlänge von N = 50 (im weiteren Verlauf mit HPDM abgekürzt) mischbar. So kann problemlos eine 5 %ige (in Tensid) Mischung, die nach 12stündiger Lagerung bei 40°C noch stabil bleibt, hergestellt werden. Ferner ist das Produkt in Siliconöl (Visk.: 100 mpa.s) trübe löslich. Eine 5 %ige Lösung separiert nach einer Lagerung bei 40°C nach 12 h.

Die Tensidschwefelgehalt-Analyse des Produktes macht das Vorliegen eines Ionenassoziats deutlich: Messungen bei pH 3, 5 und 7 ergeben kein Tensid S.

Die Elementaranalyse des Reaktionsproduktes ergibt (Element/Praxis/ Theorie): N / 1,6 % / 1,7 %; C / 45,3 % / 46,6 %; H / 9,3 % / 9,8 %; S / 3,9 % / 3,9 %; Si / 17,4 % / 20,4 %; O / n. b.

Wie nachstehend aufgeführt, zeigen Messungen wäßriger Lösungen dieser Substanz bereits bei geringen Einsatzkonzentrationen eine ausgezeichnete tensidische Aktivität, die sich in einer besonders wirksamen Oberflächenspannungserniedrigung ausdrückt.

| Konzentration (Gew.-%) | Oberflächenspannung (mN/m) (T = 25°C) |
|---|---|
| 1,0 | 20,7 |
| 0,03 | 20,9 |
| 0,01 | 20,9 |
| 0,007 | 21,0 |
| 0,002 | 23,4 |
| 0,001 | 25,6 |

Beispiel 3

Umsetzung von 3-(Glycidyloxypropyl)-1,1,1,3,5,5,5-heptamethyltrisiloxan mit (2-Hydroxypropyl)-dimethylammoniumhydrogensulfit

Die Präparation erfolgt wie schon in Beispiel 2 geschildert. Eingesetzt werden 51,58 g (0,5 Mol) (2-Hydroxypropyl)-dimethylammoniumhydrogensulfit, gelöst in 100 g Isopropanol, und 313,9 g (1 Val) 3-(Glycidyloxypropyl)-1,1,1,3,5,5,5-heptamethyltrisiloxan.

Nach der Aufarbeitung erhält man eine viskose, gelbliche Flüssigkeit [Viskosität (23°C) = 170 000 mPa.s], die sich milchig in Wasser, trübe in Siliconöl und klar in HPDM löst. Die Lösungen sind auch nach 12stündiger Lagerung bei 40°C noch stabil. Eine 1 %ige Lösung in destilliertem Wasser ergibt eine Oberflächenspannung von 21,0 mN/m und einen Spreitwert 45 mm (Polypropylen).

Beispiel 4

Umsetzung von 3-(Glycidyloxypropyl)-1,1,1,3,5,5,5-heptamethyltrisiloxan mit (2-Hydroxyethyl)-dimethylammoniumhydrogensulfit

Die Umsetzung erfolgt wie in Beispiel 2 beschrieben. Eingesetzt werden 44,57 g (0,5 Mol) (2-Hydroxyethyl)-dimethylammoniumhydrogensulfit und 313,9 g (1 Val) 3-(Glycidyloxypropyl)-1,1,1,3,5,5,5-heptamethyltrisiloxan.

Auch hier wird ein hochviskoses, klares und gelbliches Produkt erhalten [Viskosität (23°C) = 79 000 mPa.s], das sich klar in Wasser löst. Es ist weiterhin in Siliconöl (100 mPa.s) trübe und in HPDM klar löslich. Auch nach Lagerung (12 h, 40°C) ist keine Phasenseparation festzustellen. Die 1 %ige wäßrige Lösung ergibt eine Oberflächenspannung von 21,3 mN/m und eine Spreitung von 50 mm (Polypropylen).

Beispiel 5

Umsetzung von einem $\alpha,\omega$-Di-(Glycidyloxypropyl)polydimethylsiloxan einer mittleren Kettenlänge von 38 Si-Atomen mit Triethylammoniumhydrogensulfit

Experimentelles Vorgehen wie in Beispiel 2 geschildert. Es werden 45,9 g (0,25 Mol) Triethylammoniumhydrogensulfit, gelöst in 100 ml Isopropanol, und 761,9 g (0,5 Val) $\alpha,\omega$-Di-(Glycidyloxypropyl)polydimethylsiloxan mit einer mittleren Kettenlänge von 38 Si-Atomen eingesetzt.

Man erhält ein klares, farbloses Produkt [Viskosität (22°C) = 11 000 mPa.s], das sich schlecht in Wasser löst. Eine 1 %ige wäßrige Lösung ergibt eine Oberflächenspannung von 49,5 mN/m und einen Spreitwert von 7 mm (Polypropylen). Das Produkt ergibt sowohl in einem Siliconöl (Visk.: 100 mPa.s) als auch in HPDM eine klare Lösung (5 %ig),

die auch nach 12 h Lagerung bei 40°C noch stabil bleibt.

Beispiel 6

Umsetzung eines Polydimethylsiloxans einer mittleren Kettenlänge von 13 Si-Atomen und 5 seitenständig angeordneten Glycidyloxypropylgruppierungen mit Triethylammoniumhydrogensulfit

Experimentelles Vorgehen gemäß Beispiel 2. Eingesetzt werden 45,9 g (0,25 Mol) Triethylammoniumhydrogensulfit, gelöst in 100 ml Isopropanol, und 202 g (0,5 Val) des oben näher beschriebenen epoxyfunktionellen Polydimethylsiloxans.

Es wird ein klares, farbloses Produkt [ Viskosität (22°C) = 655 000 mPa.s] erhalten, das sich in Wasser, Siliconöl (Visk.: 100 mPa.s) sowie in HPDM trübe löst, was sich auch nach 12stündiger Lagerung bei 40°C nicht ändert.

Die 1 %ige wäßrige Lösung ergibt eine Oberflächenspannung von 22,4 mN/m und einen Spreitwert von 6 mm (Polypropylenfolie, keine Spreitung).

Beispiel 7

Umsetzung eines Polydimethylsiloxans einer mittleren Kettenlänge von 13 Si-Atomen, 5 seitenständig und 2 $\alpha,\omega$-ständig angeordneten Glycidyloxypropylgruppierungen mit Triethylammoniumhydrogensulfit

Analog werden 45,9 g (0,25 Mol) Triethylammoniumhydrogensulfit, gelöst in 100 ml Isopropanol, mit dem oben näher bezeichneten epoxyfunktionellen Polydimethylsiloxan zur Reaktion gebracht.

Das viskose, klare Produkt [Viskosität (24 °C) nicht meßbar] löst sich trübe in Siliconöl (Visk.: 100 mPa.s) und ebenfalls trübe in HPDM. Beide Lösungen separieren nach 12stündiger Lagerung bei 21 bzw. 40°C.

Eine 1 %ige Lösung in Wasser ergibt eine Oberflächenspannung von 24,7 mN/m und einen Spreitwert von 6 mm (keine Spreitung).

Beispiel 8

Umsetzung von 1,3-Di(glycidyloxypropyl)-1,1,3,3-tetramethyldisiloxan mit Triethylammoniumhydrogensulfit

Einsatzmengen: 91,8 g (0,5 Mol) Triethylammoniumhydrogensulfit und 190,5 g (1 Val) oben näher bezeichnetes 1,2-Diepoxydisiloxan.

Das Produkt [Viskosität (23°C) nicht meßbar] zeigt in HPDM wie auch in Siliconöl (Visk.: 100 mPa.s) gleiches Löslichkeitsverhalten wie in Beispiel 7 beschrieben.

Die Oberflächenspannung einer 1 %igen wäßrigen Lösung beträgt 23,7 mN/m, der Spreitwert 12 mm.

Beispiel 9

Umsetzung von 1,2-Epoxyoctan mit Triethylammoniumhydrogensulfit

Einsatzmengen: 183,5 g (1 Mol) Triethylammoniumhydrogensulfit, gelöst in 100 ml Isopropanol, und 246,46 g (2 Mol) 1,2-Epoxyoctan. Die Komponenten können ohne zusätzliche Lösungsmittelvariation gemäß Beispiel 2 umgesetzt werden.

Man erhält eine klare, farblose Flüssigkeit [Viskosität (23°C) = 5800 mPa.s]. Die Substanz ist in Wasser, in Siliconöl und HPDM trübe löslich. Nach 12stündiger Lagerung bei 40°C ist bei den beiden letztgenannten Lösungen eine Phasenseparation festzustellen. Eine 1 %ige Lösung in Wasser weist keine Spreitung (Polypropylenfolie) auf, erniedrigt jedoch die Oberflächenspannung auf 27 mN/m.

Beispiel 10

Umsetzung von 1,2-Epoxyhexadecan mit Triethylammoniumhydrogensulfit

Einsatzmengen: 183,5 g (1 Mol) Triethylammoniumhydrogensulfit, gelöst in 100 ml Isopropanol, und 480,86 g (2 Mol) 1,2-Epoxyhexadecan. Hier können die Komponenten ebenfalls ohne zusätzliche Lösungsmittelvariation gemäß Beispiel 2 umgesetzt werden.

Man erhält einen weißen, wachsartigen Festkörper mit einem Schmelzpunkt von 44°C. Die Oberflächenspannung der 1 %igen wäßrigen Lösung beträgt 28,2 mN/m (trübe Lösung). Eine Spreitung (7 mm) kann nicht beobachtet werden.

Die Substanz ist in Siliconöl (Visk.: 100 mPa.s) nicht, in HPDM jedoch trübe löslich.

Beispiel 11

Umsetzung von 1,2-Epoxyoctan und 3-(Glycidyloxypropyl)-1,1,1,3,5,5,5-heptamethyltrisiloxan mit Triethylammoniumhydrogensulfit

Einsatzmengen: 183,5 g (1 Mol) Triethylammoniumhydrogensulfit, gelöst in 100 ml Isopropanol, 123,23 g (1 Mol) 1,2-Epoxyoctan und 313,9 g (1 Val) 3-(Glycidyloxypropyl)-1,1,1,3,5,5,5-heptamethyltrisiloxan.

Erhalten wird eine klare, farblose Flüssigkeit [Viskosität (23°C) = 6200 mPa.s], die in Wasser und Siliconöl trübe und in HPDM gut löslich ist. Die Lösungen in Siliconöl und HPDM bleiben auch nach 12stündiger Lagerung bei 40°C stabil.

Der Spreitwert (1 % wäßrige Lösung) beträgt 35 mm und die Oberflächenspannung 20,7 mN/m.

Beispiel 12

Umsetzung von 1,2-Epoxyhexadecan und 3-(Glycidyloxypropyl)-1,1,1,3,5,5,5-heptamethyltrisiloxan mit Triethylammoniumhydrogensulfit

Einsatzmengen: 183,5 g (1 Mol) Triethylammoniumhydrogensulfit, gelöst in 100 ml Isopropanol, 240,43 g (1 Mol) 1,2-Epoxyhexadecan und 313,9 g (1 Val) 3-(Glycidyloxypropyl)-1,1,1,3,5,5,5-heptamethyltrisiloxan.

Erhalten wird eine weiße, pastöse Masse, die sich bei ca. 50°C verflüssigt und dabei etwas trübe bleibt.

Es löst sich in Wasser unvollständig, in Siliconöl (Visk.: 100 mPa.s) nicht und in HPDM trübe. Die trübe Lösung in HPDM ist auch noch nach 12 h Lagerung bei 40°C stabil.

Die 1 %ige wäßrige Lösung ergibt eine Oberflächenspannung von 22,3 mN/m und einen Spreitwert von 35 mm (Polypropylenfolie).

## Patentansprüche

1. Verfahren zur Herstellung von tensidischen Anion-Kation-Komplexen, dadurch gekennzeichnet, daß man eine kohlenstoff- oder siliciumorganische Verbindung mit mindestens einer Epoxidgruppe mit einem quaternären Ammoniumhydrogensulfit der allgemeinen Formel

$$HN^{\oplus}(R^1)_3SO_3H^{\ominus},$$

wobei

$R^1$ innerhalb des Moleküls gleich oder verschieden ist und

(1) ein Alkyl- oder Hydroxyalkylrest mit 1 bis 18 Kohlenstoffatomen,
(2) ein Phenylrest oder
(3) ein Rest der Formel $C_nH_{2n+1}CONH-(CH_2)_m-$, in der n eine Zahl von 7 bis 17 und m = 2 oder 3 ist, sein kann,

in Gegenwart eines polaren Lösungsmittels in solchen Mengen umsetzt, daß das Molverhältnis Epoxidgruppe : $HN^{\oplus}(R^1)_3SO_3H^{\ominus}$ 2 : 1 beträgt.

2. Verfahren nach Anspruch 1, dadurch gekennzeichnet, daß man als kohlenstofforganische Verbindung mit mindestens einer Epoxidgruppe eine Verbindung der allgemeinen Formel

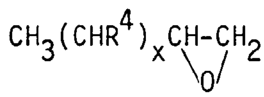 ,

wobei $R^4$ ein H- oder Alkylrest mit 2 bis 6 Kohlenstoffatomen und x eine Zahl von 2 bis 14 ist, oder das Epoxid

einer ungesättigten Fettsäure oder deren Ester verwendet.

**3.** Verfahren nach Anspruch 1, dadurch gekennzeichnet, daß man als siliciumorganische Verbindung mit mindestens einer Epoxidgruppe eine Verbindung der durchschnittlichen allgemeinen Formel

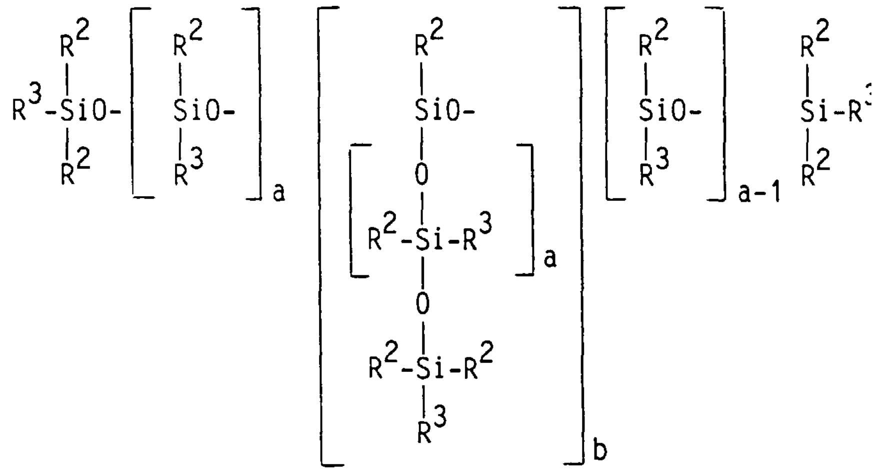

verwendet, wobei

- $R^2$ im durchschnittlichen Molekül gleich oder verschieden ist und Alkylreste mit 1 bis 18 Kohlenstoffatomen bedeutet, jedoch mindestens 90 % der Reste $R^2$ Methylreste sind,
- $R^3$ die Bedeutung des Restes $R^2$ hat oder ein über Kohlenstoff an Silicium gebundener Epoxidrest ist, mit der Maßgabe, daß im durchschnittlichen Molekül mindestens ein Rest $R^3$ ein Epoxidrest ist,
- a einen Wert von 0 bis 500 und
- b einen Wert von 0 bis 5 hat.

**4.** Verfahren nach Anspruch 3, dadurch gekennzeichnet, daß man Verbindungen verwendet, bei denen mindestens ein Rest $R^3$ ausgewählt ist aus Resten der Formeln

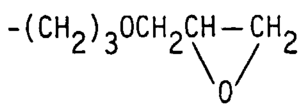
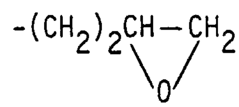

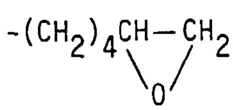
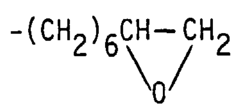

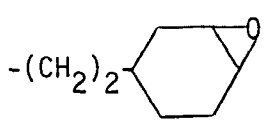
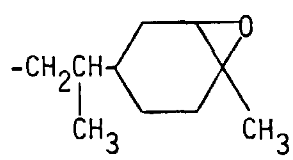
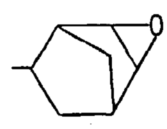

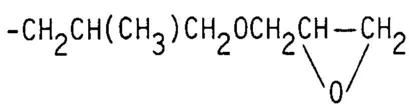

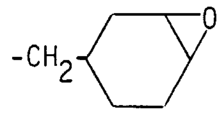

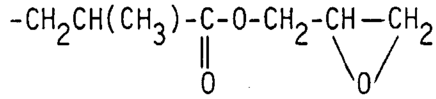

5. Verfahren nach Anspruch 1, dadurch gekennzeichnet, daß man als Edukt Gemische verschiedener siliciumorganischer Verbindungen oder Gemische von siliciumorganischen und kohlenstofforganischen Verbindungen mit jeweils mindestens einer Epoxidgruppe verwendet.

6. Verfahren nach einem oder mehreren der vorhergehenden Ansprüche, dadurch gekennzeichnet, daß man als Lösungsmittel niedrigsiedende aliphatische Alkohole verwendet.

7. Verfahren nach Anspruch 6, dadurch gekennzeichnet, daß man als Lösungsmittel Isopropanol verwendet.

## Claims

1. Process for preparing surface-active anion-cation complexes, characterized in that an organic or organosilicon compound containing at least one epoxide group is reacted with a quaternary ammonium hydrogen sulphite of the general formula

$$HN^{\oplus}(R^1)_3SO_3H^{\ominus} ,$$

where

$R^1$ are identical or different within the molecule and can each be

(1) an alkyl or hydroxyalkyl radical having from 1 to 18 carbon atoms,
(2) a phenyl radical or
(3) a radical of the formula $C_nH_{2n+1}CONH\text{-}(CH_2)_m\text{-}$, where n is a number from 7 to 17 and m = 2 or 3,

in the presence of a polar solvent in such amounts that the molar ratio of epoxide group: $HN^{\oplus}(R^1)_3SO_3H^{\ominus}$ is 2:1.

2. Process according to Claim 1, characterized in that the organic compound containing at least the one epoxide group which is used is a compound of the general formula

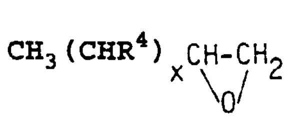 ,

where $R^4$ is H or an alkyl radical having from 2 to 6 carbon atoms and x is a number from 2 to 14, or the epoxide of an unsaturated fatty acid or an ester thereof.

3. Process according to Claim 1, characterized in that the organosilicon compound containing at least one epoxide group which is used is a compound of the average general formula

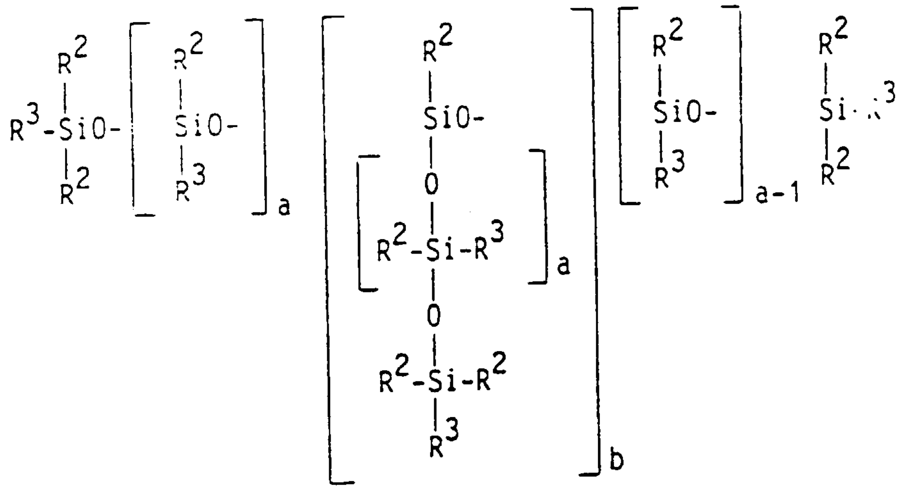

,

where

$R^2$ are identical or different in the average molecule and are alkyl radicals having from 1 to 18 carbon atoms, but at least 90% of the radicals $R^2$ are methyl radicals,

$R^3$ is as defined for the radical $R^2$ or is an epoxide radical bonded by carbon to silicon, with the proviso that in the average molecule at least one radical $R^3$ is an epoxide radical,

a has a value from 0 to 500 and

b has a value from 0 to 5.

**4.** Process according to Claim 3, characterized in that the compounds used are ones in which at least one radical $R^3$ is selected from among radicals of the formulae

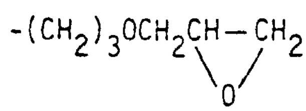

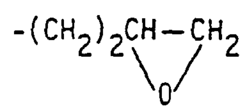

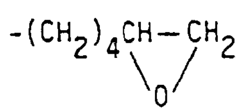

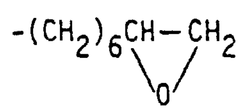

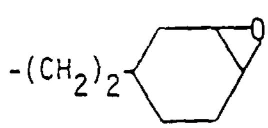

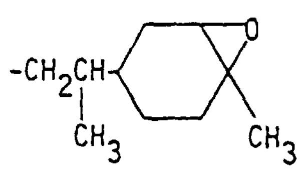

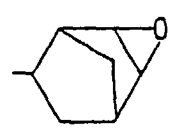

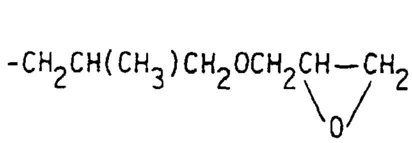

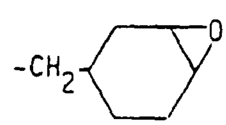

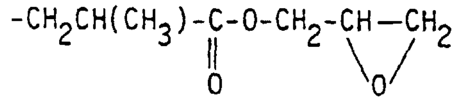

5. Process according to Claim 1, characterized in that mixtures of various organosilicon compounds or mixtures of organosilicon and organic compounds each containing at least one epoxide group are used as starting material.

6. Process according to one or more of the preceding claims, characterized in that the solvent used is a low-boiling aliphatic alcohol.

7. Process according to Claim 6, characterized in that the solvent used is isopropanol.

**Revendications**

1. Procédé de préparation de complexes tensioactifs anioniques-cationiques, caractérisé en ce qu'on fait réagir un composé organocarboné ou un composé organosilicié ayant au moins un groupe époxyde avec un hydrogénosulfite d'ammonium quaternaire, répondant à la formule générale

$$HN^{\oplus}(R^1)_3SO_3H^{\ominus},$$

où

$R^1$ sont identiques ou différents à l'intérieur de la molécule et peuvent être

(1) un reste alkyle ou hydroxyalkyle ayant de 1 à 18 atomes de carbone,
(2) un reste phényle ou
(3) un reste répondant à la formule $C_nH_{2n+1}CONH-(CH_2)_m-$, où n est un nombre de 7 à 17, et m = 2 ou 3,

en présence d'un solvant polaire, en des quantités telles que le rapport molaire groupe époxyde : $HN^{\oplus}(R^1)_3SO_3H^{\ominus}$ soit de 2 : 1.

2. Procédé selon la revendication 1, caractérisé en ce qu'on utilise, comme composé organocarboné ayant au moins un groupe époxyde, un composé répondant à la formule générale

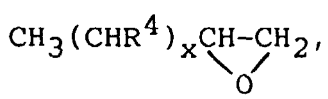

où $R^4$ est un reste d'hydrogène ou un reste alkyle ayant de 2 à 6 atomes de carbone, et x est un nombre de 2 à 14, ou l'époxyde d'un acide gras insaturé ou des esters de celui-ci.

3. Procédé selon la revendication 1, caractérisé en ce qu'on utilise, comme composé organosilicié ayant au moins un groupe époxyde, un composé répondant à la formule générale moyenne

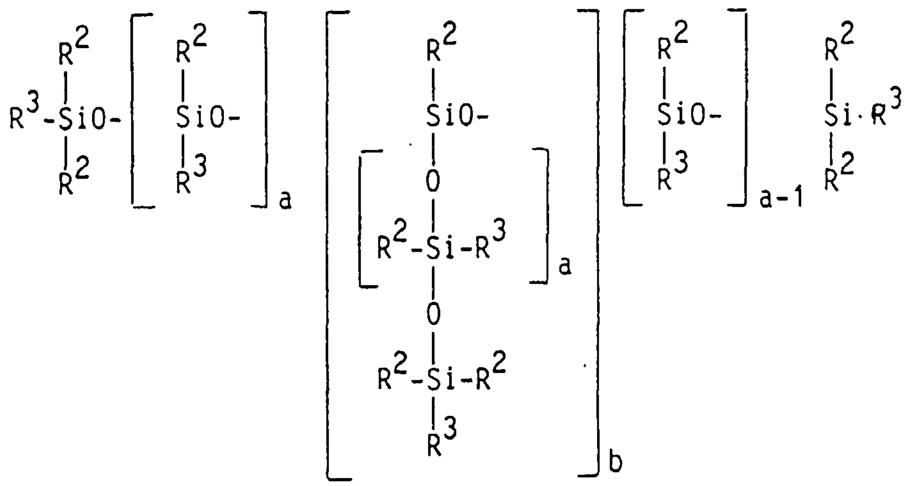

où

$R^2$ peuvent être identiques ou différents dans la molécule moyenne et signifient des restes alkyles ayant de 1 à 18 atomes de carbone, au moins 90 % des restes $R^2$ signifiant toutefois le reste méthyle,

$R^3$ a la signification du reste $R^2$ ou est un reste époxyde lié au silicium par l'intermédiaire d'un atome de carbone, avec la condition qu'au moins un reste $R^3$ soit un reste époxyde dans la molécule moyenne,

a a une valeur de 0 à 500, et

b a une valeur de 0 à 5.

**4.** Procédé selon la revendication 3, caractérisé en ce qu'on utilise des composés dans lesquels au moins un reste $R^3$ est choisi parmi les restes répondant aux formules suivantes

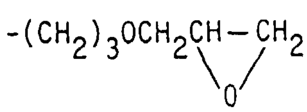

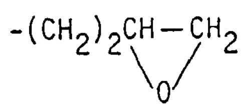

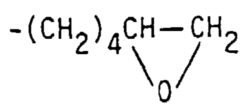

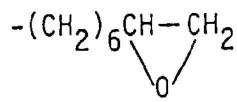

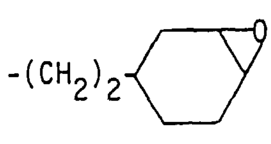

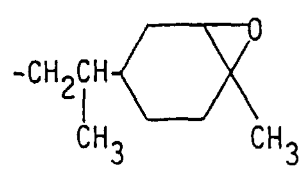

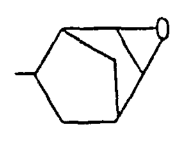

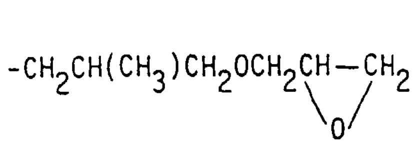

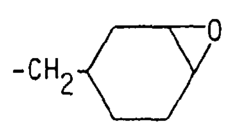

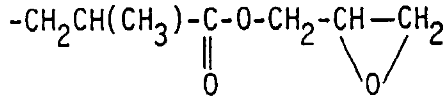

**5.** Procédé selon la revendication 1, caractérisé en ce qu'on utilise, comme produit de départ, des mélanges de divers composés organosiliciés ou des mélanges de composés organosiliciés et de composés organocarbonés ayant chacun au moins un groupe époxyde.

**6.** Procédé selon l'une ou plusieurs des revendications précédentes, caractérisé en ce qu'on utilise, comme solvant, des alcools aliphatiques à bas point d'ébullition.

**7.** Procédé selon la revendication 6, caractérisé en ce qu'on utilise l'isopropanol comme solvant.